# EUROPÄISCHE PATENTSCHRIFT

(11) **EP 2 083 750 B1**
(45) Veröffentlichungstag und Bekanntmachung des Hinweises auf die Patenterteilung: **10.03.2010**
(21) Anmeldenummer: 07817604.7
(22) Anmeldetag: 01.10.2007
(51) Int. Cl.: A61F 2/06, A61F 2/00, D04H 1/70, D04H 3/02, D04H 3/07, D06M 10/02, A61L 27/50, A61L 27/34, A61L 31/14, A61L 31/10, D06B 13/00

(54) **FLÄCHENGEFÜGE UND VERFAHREN ZUR HERSTELLUNG EINES FLÄCHENGEFÜGES**
PLANAR STRUCTURE AND METHOD FOR PRODUCING A PLANAR STRUCTURE
STRUCTURE PLANE ET PROCÉDÉ DE FABRICATION D'UNE STRUCTURE PLANE

(30) Priorität: 26.10.2006 DE 102006051013
(43) Veröffentlichungstag der Anmeldung: 05.08.2009
(73) Patentinhaber: Nonwotecc Medical Gmbh, 50829 Köln (DE)
(72) Erfinder: WILLEMS, Frank, 47441 Moers (DE)
(74) Vertreter: Castell, Klaus
(86) Internationale Anmeldenummer: PCT/DE2007/001762
(87) Internationale Veröffentlichungsnummer: WO 2008/049386

(56) Entgegenhaltungen:
- EP-A- 0 391 586
- WO-A-2004/028581
- WO-A-2004/103461
- DE-A1- 2 806 030
- DE-A1- 3 913 926
- US-A- 5 030 189

## Beschreibung

Die Erfindung betrifft ein Flächengefüge aus stellenweise miteinander verklebten Fasern.

Derartige Flächengefüge werden als Gefäßprothesen oder Gewebeflicken vor allem in der Medizintechnik verwendet.

Die Erfindung betrifft weiterhin ein Verfahren zur Herstellung eines Flächengefüges, bei dem Fasern auf einen Träger aufgebracht werden und dort zumindest teilweise verkleben. Ein derartiges Verfahren zur Herstellung von Flächengefügen ist beispielsweise aus der DE 28 06 030 bekannt. Hierbei wird eine mikroporöse feinfibrilläre Struktur erreicht, indem Polycarbonaturethane aus einer Lösung mittels einer Düse zu Mikrofasern versponnen werden. Derart hergestellte Fibrillen werden über mehrere hundert Schichten unter definierten Winkeln auf Formen aufgewickelt und an ihren Kreuzungspunkten schichtweise miteinander verschmolzen oder verklebt, so dass Gefäßprothesen oder Gewebeflicken (Patches) hergestellt werden, die eine mechanisch und biologisch stabile mikroporöse Struktur besitzen.

Die dem Blut zugewandte Innenseite der Gefäßprothese oder des Gewebeflickens soll eine möglichst fein strukturierte Oberfläche besitzen, wohingegen die Außenseite eine gröbere Oberflächenstruktur aufweisen kann, die ein sicheres Anwachsen von Bindegewebe an die Gefäßprothese nach ihrer Implantation gewährleistet.

Letztlich betrifft die Erfindung eine Gefäßprothese, die vorzugsweise nach dem erfindungsgemäßen Verfahren hergestellt ist. Es hat sich herausgestellt, dass sich mit Verfahren, wie sie in der DE 28 06 030 beschrieben sind, Gefäßprothesen herstellen lassen und nach ähnlichen Verfahren können auch Gewebeflicken, wie sogenannte Patches, hergestellt werden. Derartige Flächengefüge haben in der Regel eine mikroporöse feinfibrilläre Struktur aus biokompatiblen Materialien. Es hat sich jedoch gezeigt, dass die gefertigten Flächengefüge, die bei Gewebedefekten als Gewebeflicken oder Gefäßprothesen eingesetzt werden, nicht in dem gewünschten Maße mit dem natürlichen Gewebe kompatibel sind.

Insbesondere kleinlumige Gefäßprothesen sind bisher nicht erhältlich, da die Entwicklung derartiger Gefäßprothesen eine große Herausforderung darstellt. Alle Versuche scheiterten daran, dass die hergestellten Gefäßprothesen, hervorgerufen durch Thrombenanlagerungen und Hyperplasien, frühzeitig zu Verschleißen drohten.

In der Literatur wird zwar diskutiert, dass die Offenheitsrate wesentlich ist für die physiologische Compliance der Gefäßprothese (Salacinski et al.: "The mechanical behaivor of vascular grafts", Journal of Biomaterials Applications, Vol. 15, January 2001, Page 241 ff., sowie aus Cardiovascular Materials", Garth W. Hastings, 1991, Chapter 1, Page 1 to 16, "Mechanical Properties of Arteries and Arterial Grafts", .V. How.) In der Praxis ist jedoch kein Verfahren bekannt, das dazu geeignet ist, die für die Medizin geforderten physiologischen Eigenschaften bei künstlich hergestellten Flächengefügen bereitzustellen.

Es ist daher Aufgabe der vorliegenden Erfindung, ein Flächengefüge und ein Verfahren zur Herstellung eines derartigen Flächengefüges bereitzustellen, bei dem das Flächengefüge eine solche differenzierte natürliche Struktur besitzt, dass eine weitgehend physiologische, vorzugsweise axiale und tangentiale, Elastizität (Compliance) erreicht wird.

Diese Aufgabe wird mit einem Flächengefüge aus stellenweise miteinander verklebten Fasern gelöst, bei dem Verklebungen und/oder Fasern durch eine Ultraschallbehandlung gebrochen sind.

Der Erfindung liegt die Erkenntnis zugrunde, dass eine Ultraschallbehandlung dazu geeignet ist, die Elastizität bzw. das E-Modul einer Gefäßprothese oder eines Gewebeflickens und die Offenheitsraten am Flächengefüge zu beeinflussen. Frequenz, Intensität und Dauer der Ultraschallbehandlung müssen dabei auf den für die Herstellung des Flächengefüges verwendeten Werkstoff abgestimmt sein, um einerseits eine Veränderung der Vliesstruktur zu erreichen und andererseits eine Schädigung der Vliesstruktur zu vermeiden.

Je nach Anwendungsfall sowie Abmessungen und Dicke des Flächengefüges werden spezielle Ultraschallbehandlungsbedingungen festgelegt, damit das behandelte Flächengefüge eine weitgehend physiologische Struktur erhält.

Durch die Ultraschallbehandlung werden die Fasern bewegt, wodurch Dehnungen an den Fasern und Risse an den Fasern entstehen, die jedoch nicht nur von der Art der Ultraschallbeaufschlagung sondern auch von der Struktur des Flächengefüges und der einzelnen Fasern abhängen.

Eine vorteilhafte Ausgestaltung eines Flächengefüges sieht vor, dass die Bruchlinien statistisch verteilt ausgerichtet sind. Während bei Brüchen in Folge von Dehnungen des Flächengefüges die Bruchlinien in der Regel quer zur Dehnungsrichtung angeordnet sind, führt ein Stress durch Ultraschallbehandlung zu einer ungerichteten Dehnung, die bei mikroskopischer Betrachtung des behandelten Flächengefüges eine charakteristische Anordnung der Bruchlinien zur Folge hat.

Sowohl Gefäßprothesen als auch Gewebeflicken sind Flächengefüge mit zwei Seiten, von denen im Hinblick auf die Verwendung des Flächengefüges eine Seite die Innenseite und eine Seite die Außenseite bildet. Vorteilhaft ist es, wenn die Innenseite glatter ist als die Außenseite des Flächengefüges. Insbesondere in Verbindung mit einer speziell abgestimmten Offenheitsrate führt dies bei Gefäßprothesen dazu, dass sich auf der Gefäßprotheseninnenseite eine dünne körpereigene Neointima bildet. Die Innenseite der Flächengefüge soll somit eine möglichst feinstrukturierte Oberfläche besitzen, wohingegen die Außenseite eine gröbere Oberflächenstruktur aufweisen kann. Die gröbere Oberflächenstruktur erleichtert das Anwachsen von Bindegewebe und somit eine sichere Lage innerhalb des Körpers.

Besonders vorteilhaft ist es, wenn das Flächengefüge eine einem Gewebe angepasste axiale und tangentiale Elastizität aufweist. Es hat sich herausgestellt, dass die mit Ultraschall behandelten Gefäßprothesen oder Gewebeflicken gegenüber entsprechenden Werkstücken, die diese Behandlung nicht erfahren haben, eine bisher nicht erreichbare flexible Materialstruktur aufweisen. Außerdem haben die hergestellten Gefäßprothesen und Gewebeflicken eine dem natürlichen Gewebe entsprechende Längs- und Querelastizität.

Vorteilhaft ist es, wenn das Flächengefüge eine feinfibrilläre Struktur aufweist. Dies führt zu einer speziellen Oberflächenstruktur, die die Einlagerung von Thrombozyten in einer physiologisch vorteilhaften Menge begünstigt.

In der Praxis haben sich Flächengefüge mit Fibrillen, die einen Durchmesser von 0,5 bis 100 µ aufweisen, besonders gut bewährt. Hierbei wird vorteilhafter Weise ein Abstand von Fibrille zu Fibrille von 0,5 bis 100 µ vorgesehen.

Bei Gefäßprothesen leitet die außerordentlich hohe Compliance die Pulswellen des Blutes physiologisch im Sinne einer Windkesselfunktion fort, was in caninen Carotis- und Femoralis-Interponaten an triphasischer Flussgeschwindigkeitsamplitude erkennbar ist. In einer solchen Gefäßprothese wird vorzugsweise somit eine laminare Strömung aufrecht erhalten, so dass der bei bekannten Gefäßprothesen gefürchtete Kalibersprung vermieden wird. Außerdem werden an den Anastomosen blutschädigende Turbulenzen mit einhergehender Ablösung der Neointima, Bildung von Totwassergebieten und Hyperplasien vermieden. Die durch die Ultraschallbehandlung geschaffene Materialstruktur verleiht Gefäßprothesen eine besonders gute Formbeständigkeit für optimale Flusseigenschaften mit guter Knickstabilität bei anliegendem Innendruck.

Verfahrensmäßig wird die der Erfindung zugrunde liegende Aufgabe mit einem Verfahren zur Herstellung eines Flächengefüges gelöst, bei dem Fasern auf einem Träger aufgebracht werden und dort zumindest teilweise verkleben, wobei die verklebten Fasern mit Ultraschall behandelt werden.

Eine vorteilhafte Ausgestaltung sieht vor, dass die Fasern ein Polycarbonaturethan aufweisen. So können die Fasern insbesondere mikroporöse, feinfibrilläre Strukturen aus biokompatiblen Polycarbonaturethanen bilden. Außerdem können die Fasern ein Copolymer insbesondere eines Polycarbonaturethans aufweisen oder eine Polymerlegierung insbesondere mit Polykarbonaturethanen. Derartige Werkstoffe haben sich in der Praxis als besonders biokompatibel erwiesen und sie eignen sich sehr gut für eine Ultraschallbehandlung.

Insbesondere zur Herstellung von Gefäßprothesen wird vorgeschlagen, dass der Träger eine zylindrische Oberfläche aufweist. Entsprechend wird für die Herstellung von Gewebeflicken vorgeschlagen, dass der Träger eine plane Oberfläche aufweist.

Eine besonders vorteilhafte Ausgestaltungsvariante sieht vor, dass der Träger einen Ultraschallgenerator aufweist. Sofern die Ultraschallabgabe direkt über den Träger erfolgt, wird eine besonders intensive Beaufschlagung des Flächengefüges mit Ultraschall erreicht.

Weiterhin wird vorgeschlagen, dass die Fasern mit noch klebriger Oberfläche auf den Träger aufgebracht werden. Hierbei ist es besonders empfehlenswert, die Fasern mit einem Sprühkopf aufzubringen. Sowohl für die Herstellung von Patches als auch insbesondere für die Herstellung von Gefäßprothesen wird vorgeschlagen, dass beim Aufbringen Sprühkopf und Träger relativ zueinander bewegt werden. Beispielsweise kann der Sprühkopf um den Träger herumgeführt werden oder relativ zum Träger auf und ab bewegt werden. Eine vorteilhafte Verfahrensvariante sieht jedoch vor, dass der Träger relativ zum Sprühkopf gedreht wird und vorzugsweise dabei auch als zylindrischer Träger in Richtung seiner Längsachse bewegt wird.

Versuche haben gezeigt, dass es vorteilhaft ist, wenn die verklebten Fasern gedehnt werden. Gerade die Kombination von Dehnung der Fasern des Flächengefüges und Ultraschallbehandlung erschließt vielseitige Veränderungsmöglichkeiten, um auf die Compliance des Flächengefüges einzuwirken und vorgegebene Parameter zu erreichen.

Zur Durchführung des Verfahrens wird vorgeschlagen, dass zu einem Zylinder verklebte Fasern mit einem Dehnungsdorn gedehnt werden. Hierbei kann ein zylindrisches Flächengefüge nach dessen Herstellung auf einen Dehnungsdorn geschoben werden oder ein Dehnungsdorn wird durch das zylindrische Flächengefüge hindurch gezogen. Zur Herstellung von Patches wird vorgeschlagen, dass zu einer planen Fläche verklebte Fasern an den Rändern gehalten und über einen Dehnungsblock gedehnt werden.

Es hat sich herausgestellt, dass es vorteilhaft ist, wenn die verklebten Fasern um 5 % bis 40 %, vorzugsweise 10 % bis 30 % , gedehnt werden. Je nach Werkstofftyp des verwendeten Polycarbonaturethans und je nach Frequenz und Intensität des Ultraschalls sowie der Dehnungsparameter kommt es zu einer fast vollständigen Rückstellung der Gefäßprothese oder des Gewebeflickens oder zu einer geringfügigen bleibenden Dehnung von 3 % bis 5 %. Diese bleibende Dehnung wird nach einer Weiterbildung der Erfindung derart berücksichtigt, dass die Porengröße der Gefäßprothese oder des Gewebeflickens vor der Behandlung um ein zu erwartendes nicht rückstellendes Dehnungsmaß kleiner ausgebildet wird. Insbesondere bei Gefäßprothesen, bei denen es auf eine bestimmte Porengröße ankommt, die günstig ist für das Einwachsen von Zellen, wird bei der Herstellung die Oberflächenporengröße bewusst kleiner gestaltet, so dass sie nach der Dehnungsbehandlung auf die gewünschte Weite eingestellt ist.

Vorteilhafte Ergebnisse wurden mit einem Verfahren erzielt, bei dem die verklebten Fasern erst gedehnt und dann mit Ultraschall behandelt werden. Es hat sich gezeigt, dass die Elastizität bzw. das E-Modul einer Gefäßprothese oder eines Gewebeflickens aus biokompatiblen Polycarbonaturethanen durch die Aufdehnung mit einem Dehnungsgrad von ca. 10% bis 30 % und eine anschließende Behandlung mit Ultraschall im gedehnten Zustand wesentlich verbessert wird.

Für viele Anwendungsgebiete hat es sich als vorteilhaft erwiesen, wenn die verklebten Fasern nach der Ultraschallbehandlung ausgewaschen werden.

Eine vorteilhafte Verfahrensführung wird auch dadurch erzielt, dass die verklebten Fasern in einem Ultraschallbad behandelt werden.

Eine vorteilhafte Ausführungsform bildet eine Gefäßprothese, die vorzugsweise nach dem der vorhergehenden Verfahren hergestellt ist und einen Gefäßinnendurchmesser von weniger als 40, vorzugsweise weniger als 12 mm aufweist. Vorteilhafte Ausführungsbeispiele liegen bei 4 bis 6 mm, das heißt größer als 3 mm.

Ein Ausführungsbeispiel zur Behandlung einer Gefäßprothese und ein Ausführungsbeispiel zur Behandlung von Gewebeflicken sind in der Zeichnung dargestellt und werden nachfolgend erläutert. Es zeigt
- Fig. 1: schematisch eine auf einen Dehnungsdorn aufgebrachte Gefäßprothese,
- Fig. 2: schematisch einen in einem Rahmen aufgespannten Gewebeflicken und
- Fig. 3: einen über einem Dehnungsblock gedehnten Gewebeflicken.

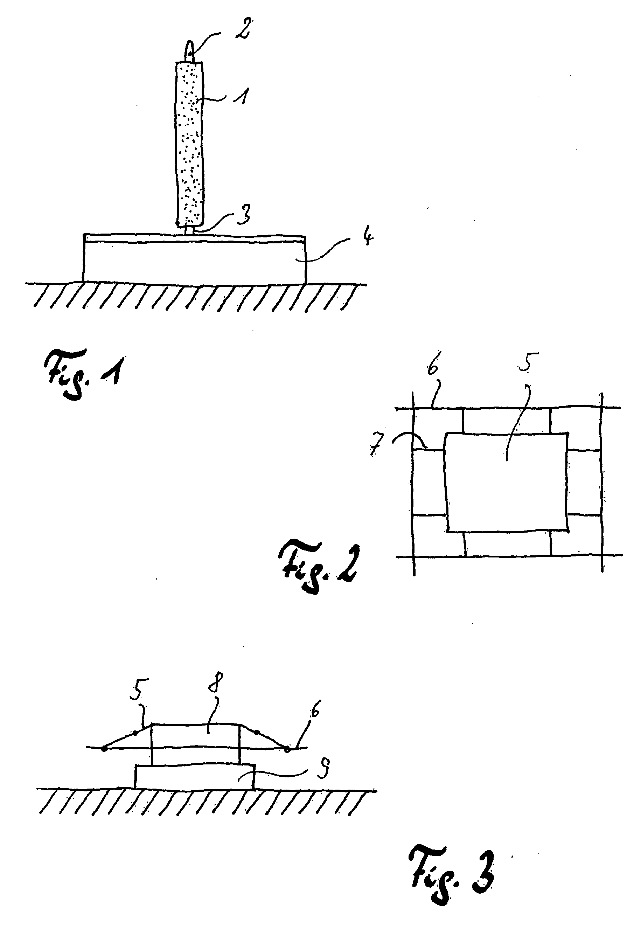

Wie in Figur 1 gezeigt, wird zur Dehnung und Behandlung einer Gefäßprothese mit Ultraschall die Gefäßprothese 1 über einen Dorn 2 gezogen, dessen Außendurchmesser ca. 10 % bis 30 % größer ist als der Innendurchmesser der zu behandelnden Gefäßprothese 1. Die Oberfläche 3 des Doms 2 besitzt eine sehr geringe Oberflächenrauhigkeit, um die Reibung an der Innenseite der Gefäßprothese 1 und damit verbundene eventuelle Schädigungen zu vermeiden.

Nach dem Aufziehen der Gefäßprothese 1 auf den Dorn 2 wird der Dorn 2 mit Ultraschall einer bestimmten Frequenz und Intensität beaufschlagt. Hierzu dient der Ultraschallgenerator 4, der im Dehnungsdorn 2 Ultraschallschwingungen erzeugt, die sich auf die Gefäßprothese 1 übertragen. Durch den Ultraschall geraten die Fibrillen (nicht gezeigt) der Gefäßprothese 1 in Schwingung. Diese Schwingungen können so stark sein, dass Fibrillen zerstört werden. Durch gezielte Einstellung der Frequenz und Intensität können alle diejenigen Fibrillen zerstört werden, die unterhalb eines bestimmten Durchmessers liegen. Dadurch können Gefäßprothesen 1 mit einer definierten Elastizität bzw. einem definierten E-Modul hergestellt werden.

Die Figuren 2 und 3 beschreiben die Behandlung von Gewebeflicken. Für die Behandlung von Gewebeflicken mit Ultraschall werden bei der Produktion hergestellte Vliesbögen 5 mittels eines rechteckigen Gestells 6 mit einem Dehnungsgrad von 10 % bis 30% beaufschlagt. Hierzu wird der Vliesbogen 5 mit Hilfe der Fäden 7 zum Gestell 6 hin gezogen. Anschließend wird der aufgespannte Bogen 5 auf einen Metallblock 8 gedrückt, der eine sehr geringe Oberflächenrauhigkeit besitzt. Hierbei wird der Rahmen 6 über dem Metallblock 8 nach unten gedrückt, so dass die Fäden 7 den Vliesbogen 5 gespannt und gedehnt halten.

Anschließend wird der Metallblock 8 mit Ultraschall einer bestimmten Frequenz und Intensität beaufschlagt. Durch den Ultraschall geraten die Fibrillen in Schwingung. Durch Einstellung einer bestimmten Frequenz und Intensität werden diejenigen Fibrillen zerstört, die unterhalb eines bestimmten Durchmessers liegen. Im Ausführungsbeispiel liegt der Ultraschallgenerator 9 unterhalb des Metallblockes 8 und überträgt über den Metallblock 8 die Ultraschallschwingungen auf den Vliesbogen 5.

Der Ultraschallgenerator kann jedoch auch gleich der Dom 2 oder der Metallblock 8 sein. Außerdem kann die Ultraschallbeaufschlagung auch in einem Ultraschallbad vorgenommen werden, bei dem eine Flüssigkeit, wie beispielsweise Wasser, die Ultraschallschwingungen zur Gefäßprothese oder zum Vliesbogen leitet.

Die Herstellung von Flächengefügen, wie Gefäßprothesen oder Vliesbögen aus polymeren Kunststoffmaterialien, ist dem Fachmann bekannt und es wird hierzu beispielsweise auf die bereits zuvor genannte Offenlegungsschrift DE 280 60 30 voll inhaltlich Bezug genommen. Im Ausführungsbeispiel wird ein, gemäß einem in dieser Offenlegungsschrift beschriebenen Verfahren, hergestelltes Flächengefüge mit Ultraschall behandelt, um ein Flächengefüge mit einer vorbestimmten physiologischen Compliance herzustellen. Hierbei wird eine gelartige Flüssigkeit aus gelöstem Granulat versprüht. Beim Versprühen verdampft das Lösungsmittel, dessen Siedepunkt unter 100 °C liegt, und es entstehen Fasern, die mit Abstand nebeneinander in Schichten übereinander abgelegt werden. Ein fertiges Flächengefüge hat zwischen 50 und 300 Schichten.

## Patentansprüche

1. Gefäßprothese aus stellenweise miteinander verklebten Fibrillen eines Polycarbonaturethans, eines Copolymers eines Polycarbonaturethans oder einer Polymerlegierung mit Polycarbonaturethanen, ***dadurch gekennzeichnet, dass*** die Fibrillen mit einen Durchmesser von 0,5 bis 100 µm und einen Abstand von 0,5 bis 100 µm aufweisen Verklebungen und/oder Fasern durch eine Ultraschallbehandlung gebrochen sind und die Bruchlinien statistisch verteilt ausgerichtet sind, wobei die mikroporöse feinfibrilläre Struktur eine einem Gewebe angepasste axiale und tangentiale Elastizität eine dem natürlichen Gewebe entsprechende Längs- und Querelastizität aufweist und auf der Außenseite der Gefäßprothese eine gröbere Struktur als auf der den Blut zugewandten Innenseite besitzt

2. Gefäßprothese nach Anspruch 1, ***dadurch gekennzeichnet, dass*** sie einen Gefäßinnendurchmesser von weniger als 12 mm aufweist.

3. Verfahren zur Herstellung eines Flächengefüges insbesondere nach Anspruch 1 oder 2, bei dem Fasern auf einen Träger aufgebracht werden und dort zumindest teilweise verkleben, ***dadurch gekennzeichnet, dass*** die verklebten Fasern mit Ultraschall behandelt werden, die Fasern mit noch klebriger Oberfläche mit einem Sprühkopf, welcher um den Träger herumgeführt wird und sich relativ zum Träger auf und ab bewegt, auf den Träger aufgebracht werden, die verklebten Fasern um 5 % bis 40 % gedehnt werden und nach der Ultraschallbehandlung ausgewaschen werden.

4. Verfahren nach Anspruch 3, ***dadurch gekennzeichnet, dass*** die Fasern ein Polykarbonaturethan aufweisen.

5. Verfahren nach Anspruch 3 oder 4, ***dadurch gekennzeichnet, dass*** die Fasern ein Copolymer insbesondere eines Polycarbonaturethans aufweisen.

6. Verfahren nach einem der Ansprüche 3 bis 5, ***dadurch gekennzeichnet, dass*** die Fasern eine Polymerlegierung insbesondere mit einem Polycarbonaturethan aufweisen.

7. Verfahren nach einem der Ansprüche 3 bis 6, ***dadurch gekennzeichnet, dass*** der Träger eine zylindrische Oberfläche aufweist.

8. Verfahren nach einem der Ansprüche 3 bis 6, ***dadurch gekennzeichnet, dass*** der Träger eine plane Oberfläche aufweist.

9. Verfahren nach einem der Ansprüche 3 bis 8, ***dadurch gekennzeichnet, dass*** der Träger einen Ultraschallgenerator aufweist.

10. Verfahren nach einem der Ansprüche 3 bis 9, ***dadurch gekennzeichnet, dass*** zu einem Zylinder verklebte Fasern mit einem Dehnungsdorn gedehnt werden.

11. Verfahren nach einem der Ansprüche 3 bis 10, ***dadurch gekennzeichnet, dass*** zu einer planen Fläche verklebte Fasern an den Rändern gehalten und über einem Dehnungsblock gedehnt werden.

12. Verfahren nach einem der Ansprüche 3 bis 11, ***dadurch gekennzeichnet, dass*** die verklebten Fasern in einem Ultraschallbad behandelt werden.

## Claims

1. A vascular prosthesis of fibrils of a polycarbonate urethane, a copolymer of a polycarbonate urethane or a polymer alloy with polycarbonate urethanes bonded together in spots, **characterized in that** the fibrils with a diameter of 0.5 to 100 µm and a spacing of 0.5 to 100 µm having adhesive bondings and/or fibers, which are broken by an ultrasonic treatment, the fracture lines being randomly oriented, wherein the microporous fine fibrillar structure has an axial and tangential elasticity adapted to the tissue, a longitudinal and transverse elasticity corresponding to that of natural tissue and a coarser structure on the outside of the vascular prosthesis than on the inside facing the blood.

2. The vascular prosthesis according to claim 1, **characterized in that** it has a vascular inside diameter of less than 12 mm.

3. The method for producing a planar structure, in particular according to claim 1 or 2, in which fibers are applied to a backing and at least partially bonded there, **characterized in that** the bonded fibers are treated with ultrasound; while the surface is still tacky, the fibers are applied to the backing with a spray head, which is guided around the backing and moves up and down relative to the backing; the bonded fibers are stretched by 5% to 40% and are washed out after the ultrasonic treatment.

4. The method according to claim 3, **characterized in that** the fibers have a polycarbonate urethane.

5. The method according to claim 3 or 4, **characterized in that** the fibers have a copolymer, in particular a polyurethane.

6. The method according to any one of claims 3 to 5, **characterized in that** the fibers have a polymer alloy with a polycarbonate urethane in particular.

7. The method according to any one of claims 3 to 6, **characterized in that** the backing has a cylindrical surface.

8. The method according to any one of claims 3 to 6, **characterized in that** the backing has a planar surface.

9. The method according to any one of claims 3 to 8, **characterized in that** the backing has an ultrasonic generator.

10. The method according to any one of claims 3 to 9, **characterized in that** fibers bonded to form a cylinder are stretched with a stretching mandrel.

11. The method according to any one of claims 3 to 10, **characterized in that** fibers bonded to form a planar surface are held at the edges and stretched over a stretching block.

12. The method according to any one of claims 3 to 11, **characterized in that** the bonded fibers are treated in an ultrasonic bath.

## Revendications

1. Prothèse vasculaire composée de fibrilles collées ensemble de polycarbonaturéthane, de copolymère de polycarbonaturéthane ou d'un alliage de polymère contenant du polycarbonaturéthane, **caractérisée en ce que** les fibrilles présentant un diamètre de 0,5 à 100 µm, avec des collages et/ou fibres espacés de 0,5 à 100 µm, sont coupées par un traitement aux ultrasons et les lignes de coupure sont orientées en répartition statistique, la structure microporeuse à fibrilles fines présentant une élasticité axiale et tangentielle adaptée à un tissu et une élasticité longitudinale et transversale correspondant au tissu naturel et possédant sur la face extérieure de la prothèse vasculaire une structure plus grossière que sur la face intérieure tournée vers le sang.

2. Prothèse vasculaire selon la revendication 1, **caractérisée en ce qu'**elle présente un diamètre intérieur de vaisseau de moins de 12 mm.

3. Procédé de fabrication d'une structure surfacique en particulier selon la revendication 1 ou 2, dans lequel des fibres sont appliquées sur un support et y sont collées au moins partiellement, **caractérisé en ce que** les fibres collées sont traitées aux ultrasons, les fibres à surface encore collante sont appliquées sur le support à l'aide d'une tête de pulvérisation qui est guidée autour du support et monte et descend par rapport au support, les fibres collées sont étirées de 5 % à 40 % et lavées après le traitement aux ultrasons.

4. Procédé selon la revendication 3, **caractérisé en ce que** les fibres contiennent un polycarbonaturéthane.

5. Procédé selon la revendication 3 ou 4, **caractérisé en ce que** les fibres contiennent un copolymère, en particulier de polycarbonaturéthane.

6. Procédé selon une des revendications 3 à 5, **caractérisé en ce que** les fibres contiennent un alliage de polymère, en particulier contenant un polycarbonaturéthane.

7. Procédé selon une des revendications 3 à 6, **caractérisé en ce que** le support présente une surface cylindrique.

8. Procédé selon une des revendications 3 à 6, **caractérisé en ce que** le support présente une surface plane.

9. Procédé selon une des revendications 3 à 8, **caractérisé en ce que** le support présente un générateur d'ultrasons.

10. Procédé selon une des revendications 3 à 9, **caractérisé en ce que** les fibres collées pour former un cylindre sont étirées avec une forme d'étirement.

11. Procédé selon une des revendications 3 à 10, **caractérisé en ce que** les fibres collées pour former une surface plane sont maintenues par les bords et étirées au dessus d'un bloc d'étirement.

12. Procédé selon une des revendications 3 à 11, **caractérisé en ce que** les fibres collées sont traitées dans un bain d'ultrasons.
